# FASCICULE DE BREVET EUROPEEN

(11) **EP 0 711 539 B1**
(45) Date de publication et mention de la délivrance du brevet: **25.08.1999**
(21) Numéro de dépôt: 95402212.5
(22) Date de dépôt: 03.10.1995
(51) Int. Cl.: A61K 7/00

(54) **Utilisation de dérivé de l'acide salicylique comme stabilisant d'une émulsion huile-dans-eau, exempté de tensioactif, exempte de tensioactif**
Verwendung eines Salicylsäurederivats als Stabilisator für eine Öl-in-Wasser-Emulsion, die keinen grenzflächenaktiven Stoff enthält
Use of a salicylic acid derivative as stabilizer of oil-in-water emulsion, surfactants-free

(30) Priorité: 03.11.1994 FR 9413127
(43) Date de publication de la demande: 15.05.1996
(73) Titulaire: L'OREAL, 75008 Paris (FR)
(72) Inventeur: Collin, Nathalie,, F-92330 Sceaux (FR); Quemin, Eric, F-93420 Villepinte (FR); Candau, Didier, F-91570 Bievres (FR)
(74) Mandataire: Lhoste, Catherine

(56) Documents cités:
- EP-A- 0 378 936
- EP-A- 0 570 230
- EP-A- 0 585 170
- EP-A- 0 616 799
- FR-A- 2 607 498
- GB-A- 2 174 906

## Description

La présente invention se rapporte à l'utilisation d'un dérivé de l'acide salicylique comme stabilisant d'une émulsion huile-dans-eau ainsi qu'à des émulsions cosmétiques et/ou dermatologiques contenant ce dérivé. Ces émulsions sont notamment destinées au traitement ou au soin de la peau aussi bien du visage que du corps humain, y compris le cuir chevelu et les ongles et encore plus spécialement au traitement et/ou lutte contre l'acné, les peaux grasses à tendance acnéïque, le vieillissement (rides, ridules, teint), et la pigmentation de la peau.

Il est connu d'utiliser des dérivés de l'acide salicylique comme agent kératolytique pour traiter l'acné et comme agent d'antivieillissement dans des compositions cosmétiques et/ou dermatologiques (voir FR-A-2581542 et EP-A-378936).

Ces dérivés sont d'un grand intérêt, étant donné leurs effets biologiques sur la peau. Ils permettent notamment de conférer au visage un teint lumineux et éclatant et donc une bonne mine, un aspect lisse et plus jeune, ainsi que de faire disparaître les comédons dûs à l'acné.

Cependant, leur utilisation pose un problème dans la mesure où ils se présentent sous forme cristalline et où ils ne sont solubles ni dans l'eau ni dans les huiles traditionnellement utilisées dans les domaines cosmétique et dermatologique comme les huiles minérales (vaseline, paraffine).

Ainsi, si on les introduit tels quels dans les compositions cosmétiques et/ou dermatologiques, ils restent à l'état de cristaux, ce qui rend l'utilisation de la composition les contenant, inefficace pour le traitement de la peau.

Ces dérivés sont, en revanche, solubles dans les alcools inférieurs comme l'éthanol ou l'isopropanol, les alcools de Guerbet ou dans des solvants tels que l'octyldodécanol, certains glycols, les alcools gras à chaîne courte (< C12), polyoxyéthylénés ou polyoxypropylénés ou encore les esters à chaîne courte (<C12).

Les alcools inférieurs présentent l'inconvénient de dessécher et d'irriter la peau ; ils sont mal tolérés par les peaux sensibles ou fragiles surtout en applications répétées. On préfère donc éviter leur utilisation dans les produits de soin du corps et/ou du visage.

Par ailleurs, les alcool gras et les esters gras à chaîne courte ainsi que certains glycols permettant la solubilisation de ces dérivés entraînent la pénétration en profondeur d'actifs dans la peau, ce qui n'est pas forcément souhaitable pour des produits de soin.

Actuellement, on cherche de manière générale à limiter de plus en plus l'utilisation de solvants dans les produits de soin pour la peau, ces solvants n'étant pas toujours bien tolérés et pouvant entraîner des irritations quand on les utilise en trop grande quantité.

La demanderesse a donc cherché à formuler les dérivés de l'acide salicylique dans des compositions contenant le moins possible de solvants.

Par ailleurs, ces dérivés présentent l'inconvénient majeur de provoquer des picotements, des démangeaisons, des tiraillements après leur application, pouvant conduire à un inconfort tel que leur utilisation par des personnes à peau sensible est souvent rédhibitoire. Cet inconfort est dû notamment à la fonction acide de ces dérivés.

Aussi, il subsiste le besoin d'une composition cosmétique et/ou dermatologique à base de dérivé de l'acide salicylique, conférant à la peau cette bonne mine et ce rajeunissement ainsi que l'élimination des comédons, sans entraîner les inconvénients ci-dessus.

La demanderesse a notamment trouvé, de manière surprenante, que les dérivés de l'acide salicylique pouvaient être introduits sous forme salifiée dans des compositions cosmétiques et/ou dermatologiques, sans que se produise la recristallisation de ces dérivés et sans qu'il soit nécessaire d'utiliser une grande quantité de solvant.

De façon surprenante, la demanderesse a découvert qu'il était possible de stabiliser des émulsions huile-dans-eau par des sels de dérivés de l'acide salicylique permettant de s'affranchir de l'emploi de solvants lipophiles irritants, sans se heurter à une recristallisation de ces dérivés.

Ainsi, ce ou ces dérivés se placent à l'interface de l'huile et de l'eau et entourent les gouttelettes d'huile. Ils stabilisent ainsi l'émulsion obtenue sans que l'emploi de tensioactif s'avère nécessaire.

De façon plus précise, l'invention a pour objet l'utilisation d'au moins un dérivé de l'acide salicylique salifié par une base comme agent de dispersion d'une phase huileuse dans une phase aqueuse, pour obtenir une émulsion huile-dans-eau, exempte de tensioactif, ce dérivé étant situé à l'interface huile/eau.

Ces dérivés salifiés présentent, en outre, l'avantage d'être moins agressifs que leurs correspondants acides tout en ayant des propriétés comparables.

En particulier, les dérivés de l'acide salicylique auxquels s'appliquent l'invention présentent la formule (1) suivante : 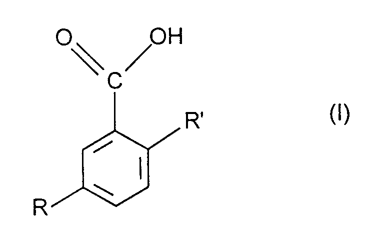 dans laquelle :
R représente une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 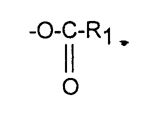 où R₁ est un groupement aliphatique saturé ou insaturé ayant de 1 à 18 atomes de carbone.

Cette émulsion peut être utilisée dans tous les domaines utilisant ce type de forme galénique et notamment dans les domaines cosmétique et pharmaceutique.

Aussi, l'invention a encore pour objet une émulsion cosmétique et/ou dermatologique, contenant au moins un dérivé de l'acide salicylique salifié présentant avantageusement la formule (I) ci-dessus, ce dérivé étant situé à l'interface huile/eau.

De préférence, le radical R comporte au moins 4 atomes de carbone. Il est par exemple formé d'un radical alkyle ou alcoxy linéaire saturé ayant de 4 à 11 atomes de carbone.

De façon avantageuse, le dérivé de l'acide salicylique est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, salifiés par une base. On peut cependant utiliser les sels des acides 5-tert-octylsalicylique, 3-tert-butyl-5 ou 6-méthylsalicylique, 3,5-diisopropylsalicylique, 5-butoxysalicylique, 5-octyloxysalicylique. On peut aussi utiliser ceux décrits dans le document EP-A-570230.

Comme base susceptible de salifier le dérivé de l'acide salicylique, on peut citer les bases minérales comme les hydroxydes de métaux alcalins (hydroxydes de sodium et de potassium) ou les hydroxydes d'ammonium ou mieux encore les bases organiques.

Contrairement à l'enseignement du brevet US-A-5 091 171, la demanderesse a trouvé que les dérivés de l'acide salicylique salifiés avaient des propriétés comparables à celles du dérivé de l'acide salicylique acide correspondant et ce, quelque soit la base utilisée pour la salification (y compris les hydroxydes alcalins).

De préférence, on utilise des bases amphotères pour la salification des dérivés de l'acide salicylique, c'est-à-dire des bases ayant à la fois des groupements fonctionnels anioniques et cationiques.

Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine. Ces bases sont utilisées en quantités suffisantes pour amener le pH de l'émulsion entre 5 et 7 et donc proche de celui de la peau. Il s'ensuit une grande compatibilité de l'émulsion de l'invention vis-à-vis de la peau.

De préférence, la base est l'arginine ou mieux la lysine. Cette dernière permet l'obtention d'une émulsion très fine stable au moins 2 mois à température ambiante.

A titre d'exemple de dérivé de l'acide salicylique salifié utilisable dans l'invention, on peut citer le n-dodécanoyl-5 salicylate de N,N-diméthyl N-(hydroxy-2 éthyl) ammonium noté DSDHA, le n-octanoyl-5 salicylate d'hexadécyl-triméthyl ammonium et de façon générale tous les dérivés aminés cités dans le document FR-A-2 607 498. On peut aussi utiliser ceux décrits dans le document EP-A-36534.

Selon l'invention, le ou les dérivés de l'acide salicylique salifiés peuvent être utilisés en quantité suffisante pour assurer la stabilisation de l'huile ainsi que sa dispersion dans la phase aqueuse. En pratique, on utilise de 0,1 % à 10 % en poids de dérivé par rapport au poids total de l'émulsion et de préférence de 1 % à 5 %.

Les émulsions de l'invention peuvent contenir une ou plusieurs huiles classiquement utilisées dans les domaines cosmétique et dermatologique. En particulier, on peut utiliser une huile végétale (tournesol, amande douce, pépin de cassis, abricot), minérale (vaseline), siliconée (cyclométhicone à 5 ou 6 groupements Si-O) ou fluorée (perfluoropolyéther).

De façon avantageuse, on utilise une ou plusieurs huiles polaires et par exemple une huile choisie parmi le Miglyol, les huiles synthétiques (huile de purcellin, alcools, esters ou acides gras comme les triglycérides, le palmitate ou myristate d'octyle, les esters ou éthers hydroxylés, oxyéthylénés ou oxypropylénés comme le lactate d'isostéaryle ou de myristyle).

L'huile ou les huiles de l'invention peuvent être utilisées à raison de 10 % à 70 % du poids total de l'émulsion et de préférence de 20 % à 40 %.

Il est possible d'introduire dans l'émulsion de l'invention un ou plusieurs autres constituants classiquement utilisés dans les domaines considérés tels que les antioxydants (vitamine E), les conservateurs, les opacifiants, les colorants, les pigments (oxydes de titane ou de zinc), les parfums, les charges, les tensioactifs (diméthiconol) ainsi que des adjuvants lipophiles tels que des huiles ou acides gras essentiels, des céramides, des pseudocéramides, des glycocéramides. Ces adjuvants peuvent représenter, au total, de 0,1 % à 15 % du poids total de l'émulsion.

L'émulsion de l'invention peut, en outre, contenir un ou plusieurs gélifiants comme les argiles, les gommes polysaccharides (xanthane), les polymères carboxyvinyliques ou carbomers. Ces gélifiants sont utilisés de préférence à des concentrations allant de 0,1 % à 10 % du poids total de la composition.

L'émulsion de l'invention peut, en outre, contenir un ou plusieurs actifs lipophiles ou hydrophiles différents des dérivés de l'acide salicylique salifiés utilisés comme stabilisant de l'émulsion. Ces actifs peuvent être des agents hydratants et/ou cicatrisants (glycérine et allantoïne ainsi que leurs dérivés et les compositions les contenant), des β-hydroxyacides et notamment l'acide salicylique et ses dérivés non salifiés, les α-hydroxyacides comme l'acide glycolique, tartrique etc..., des filtres hydrophiles ou lipophiles pour filtrer les rayons visibles et/ou ultraviolets ainsi que des actifs dermatologiques. Ces actifs sont employés selon les quantités classiquement utilisées dans les domaines considérés et notamment à raison de 0,05 % à 5 % du poids total de l'émulsion.

De façon avantageuse, les émulsions de l'invention sont exemptes de solvant et/ou exemptes d'agent émulsionnant. Ceci va aussi dans le sens d'une émulsion peu agressive et non irritante susceptible d'être utilisée par des personnes à peau sensible.

L'invention a encore pour objet l'utilisation de l'émulsion ci-dessus pour le traitement non thérapeutique de la peau, par application topique.

L'invention a aussi pour objet l'utilisation de l'émulsion ci-dessus pour prévenir et/ou lutter contre l'acné et/ou les peaux grasses et/ou le vieillissement et/ou la pigmentation de la peau, de façon non thérapeutique.

L'invention a encore pour objet un procédé de traitement cosmétique de la peau pour lutter contre l'acné et/ou les peaux grasses et/ou le vieillissement et/ou la pigmentation de la peau, consistant à appliquer sur celle-ci l'émulsion définie ci-dessus.

La description qui suit est donnée à titre illustratif et non limitatif. Dans les exemples le pourcentage est donné en poids.

### Exemple 1

| | |
|---|---|
| Acide n-octanoyl-5-salicylique | 5 % |
| Lysine | 1 équivalent molaire |
| Mygliol 812 | 70 % |
| Conservateurs | 0,3 % |
| Eau permutée | qsp 100 % |

Cette émulsion est assez compacte et de toucher légèrement gras. Elle se présente sous forme d'un onguent pour le traitement des peaux très sèches.

### Exemple 2 : Crème anti-âge pour le visage

| | |
|---|---|
| Acide n-octanoyl-5-salicylique | 1,5 % |
| Lysine | 1,6 % |
| Huile végétale | 22 % |
| Béhénate de glycéryle (corps gras) | 1 % |
| Huile de purcellin | 2 % |
| Fraction liquide de beurre de karité | 3 % |
| Cyclométhicone D5 | 5 % |
| Gomme de xanthane | 0,3 % |
| Glycérine | 3 % |
| Conservateurs | 0,7 % |
| Antioxydant | 0,05 % |
| Eau permutée | qsp 100 % |

En appliquant cette crème légère sur le visage quotidiennement, les fines ridules s'estompent, le teint devient éclatant et la peau est lissée au cours du traitement.

### Exemple 3 : Crème anti-âge pour le corps

| | |
|---|---|
| Acide n-octanoyl-5-salicylique | 2,5 % |
| Lysine | 2,13 % |
| Mygliol 812 | 35 % |
| Carbomer | 0,75 % |
| Conservateurs | 0,3 % |
| Eau permutée | qsp 100 % |

Cette crème souple appliquée quotidiennement sur le corps a pour effet un éclaircissement et un lissage de la peau très adoucie par le traitement.

### Exemple 4 : Crème anti-âge pour le visage

| | |
|---|---|
| Acide n-octanyol-5 salicylique | 1 % |
| Lysine | 1,3 % |
| N-oleyl di-hydrosphingosine | 0,1 % |
| Huile d'amandes d'abricot | 20 % |
| Fraction liquide de beurre de karité | 8 % |
| Mélange d'éthyl-2 hexanoate de cétylstéaryle, myristate d'iso-propyle (90/10) (corps gras) | 4 % |
| Conservateurs | 0,6 % |
| Antioxydant | 0,05 % |
| Carbomer | 0,75 % |
| Gomme de xanthane | 0,3 % |
| Glycérine | 5 % |
| Mélange d'alcools gras (Stéarylique-Octyldodécanol-Behenylique 40/10/50) | 1 % |
| Eau permutée | qsp 100 % |

La demanderesse a testé l'émulsion de l'exemple 4 selon l'invention, pour le traitement du vieillissement sur un panel de 10 femmes. L'émulsion de l'exemple 4 a été considérée comme aussi efficace qu'une émulsion contenant de l'acide n-octanoyl-5-salicylique non salifié, toute chose égale par ailleurs, avec en plus une absence d'irritation.

Par ailleurs, la demanderesse a testé la desquamation provoquée par l'émulsion de l'exemple 4, comparativement à la même formule sans lysine, en utilisant une turbine à ailettes.

La turbine à ailette permet de mesurer la desquamation spontanée. Cette turbine comprend une chambre, et une ailette placée dans cette chambre. La turbine est placée sur la surface de prélèvement, par exemple le bras du testeur de sorte que l'ailette ne touche pas cette surface on introduit dans la chambre de la turbine 0,3 ml d'un tampon phosphate et, pendant 1 mn, on fait tourner l'ailette, qui va agiter le tampon phosphate.

Les cornéocytes prêts à desquamer se détachent spontanément de la peau et se retrouvent dans le tampon phosphate. On récupère ensuite dans un tube en verre le tampon contenant les cornéocytes ainsi prélevés et on centrifuge le tube pendant 10 mn à 4000 tours par minute. On retire le surnageant pour conserver seulement 1 ml de suspension. Les cornéocytes sont colorés (colorant = 1 volume de Fuschine basique à 1 % + 1 volume de cristal violet à 1 %) et comptés sous microscope. Le nombre de cornéocytes comptés est d'autant plus important que le produit appliqué auparavant sur la surface de prélèvement favorise la desquamation.

Le test est réalisé à T₀ et T₂₄ₕ, c'est-à-dire au moment de l'application de la crème et 24 heures après application.

Le résultat du test indiqué ci-dessous montre que l'émulsion de l'exemple 4 provoque une desquamation supérieure à celle de l'émulsion sans lysine, et a donc un effet kératolytique plus important.

| | T₀ | T₂₄ₕ |
|---|---|---|
| Emulsion de l'exemple 4 | 63,5 | 173,6 |
| Emulsion sans lysine | 60,9 | 67,5 |
| Peau nue | 67,5 | 66,5 |

On montre dans le tableau ci-après l'effet de la dose de l'émulsionnant de l'invention sur la stabilité de l'émulsion. La composition étudiée est la suivante :

| | |
|---|---|
| Acide n-octanoyl-5-salicylique | x % |
| Lysine | y % |
| Mygliol 812 | 34,85 % |
| Carbomer | 0,75 % |
| Conservateurs | 0,7 % |
| Eau permutée | qsp 100 % |

| | |
|---|---|
| y = 1 équivalent molaire pour salifier x + ce qu'il faut pour neutraliser le carbomer. | |

**TABLEAU**

| Dose x | Dose y | Résultat (T₀) | viscosité (T₀) | pH (T₀) |
|---|---|---|---|---|
| 0,1 | 0,8 | casse après ¼ d'heure | - | - |
| 0,5 | 1,03 | fine-quelques relargages huile près des bords | 72 poises | 5,89 |
| 1 | 1,3 | très fine | 40 poises | 6,07 |
| 1,5 | 1,58 | très fine | 36 poises | 5,82 |
| 2,5 | 2,13 | très fine | 20 poises | 5,83 |
| 5 | 3,51 | très fine-pas de recristallisation à T₁₅ | 8,6 poises | 5,78 |

Par très fine, il faut comprendre une émulsion ayant des gouttelettes d'huile de 0,3µm à 0,5µm.

D'après ce tableau on constate que l'émulsion est stable, sans cristaux d'acide n-octanoyl-5-salicylique, et présente de fines, voire très fines gouttelettes d'huile dispersées dans l'eau pour des quantités de dérivé de l'acide salicylique allant de 0,1 % à 5 % en poids, au temps initial T₀. Pour une stabilité de plusieurs jours, il est souhaitable d'utiliser de 1 % à 5 % en poids de dérivé de l'acide salicylique comme émulsionnant.

La demanderesse a donc bien trouvé un moyen efficace de stabiliser les dérivés de l'acide salicylique tout en supprimant les effets secondaires irritants des compositions de l'art antérieur contenant ces dérivés sous forme acide. Ainsi, il est maintenant possible aux personnes à peau sensible d'utiliser des compositions contenant ces dérivés pour lutter et/ou prévenir le vieillissement, la peau grasse, l'acné et la pigmentation de la peau.

## Revendications

1. Utilisation d'un dérivé de l'acide salicylique salifié par une base comme agent de dispersion d'une phase huileuse dans une phase aqueuse pour obtenir une émulsion huile-dans-eau, exempte de tensioactif, ce dérivé étant situé à l'interface huile/eau.

2. Utilisation selon la revendication 1, caractérisée en ce que le dérivé présente la formule (I) suivante : 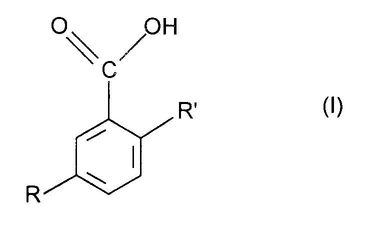 dans laquelle :
R représente une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 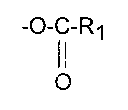 où R₁ est un groupement aliphatique saturé ou insaturé ayant de 1 à 18 atomes de carbone.

3. Utilisation selon la revendication 2, caractérisée en ce que le radical R est un radical alkyle ou alcoxy ayant de 4 à 11 atomes de carbone.

4. Utilisation selon l'une quelconque des revendications précédentes, caractérisée en ce que le dérivé de l'acide salicylique est l'acide n-octanoyl-5-salicylique.

5. Utilisation selon l'une quelconque des revendications précédentes, caractérisée en ce que la base est une base organique.

6. Utilisation selon l'une quelconque des revendications précédentes, caractérisée en ce que la base est une base amphotère.

7. Utilisation selon l'une quelconque des revendications précédentes, caractérisée en ce que la base est choisie parmi les acides aminés.

8. Utilisation selon l'une quelconque des revendications précédentes, caractérisée en ce que la base est choisie parmi la lysine ou l'arginine.

9. Emulsion cosmétique et/ou dermatologique, caractérisée en ce qu'elle contient au moins un dérivé d'acide salicylique de formule (I) ci-après, salifié par une base : 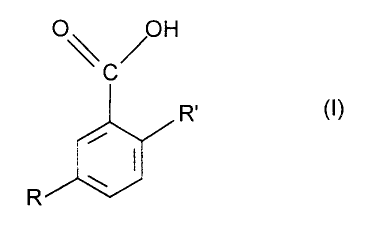 dans laquelle :
R représente une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone;
R' représente un groupement hydroxyle ou une fonction ester de formule : 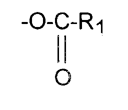 où R₁ est un groupement aliphatique saturé ou insaturé ayant de 1 à 18 atomes de carbone,
ce dérivé étant situé à l'interface huile/eau.

10. Emulsion selon la revendication 9, caracterisée en ce qu'elle est exempte de tensioactif.

11. Emulsion selon la revendication 9 ou 10, caractérisée en ce que le radical R est un radical alkyle ou alcoxy ayant de 4 à 11 atomes de carbone.

12. Emulsion selon l'une des revendications 9 à 11, caractérisée en ce que le dérivé de l'acide salicylique est l'acide n-octanoyl-5-salicylique.

13. Emulsion selon l'une des revendications 9 à 12, caractérisée en ce que la base est une base organique.

14. Emulsion selon l'une quelconque des revendications 9 à 13, caractérisée en ce que la base est une base amphotère.

15. Emulsion selon l'une quelconque des revendications 9 à 14, caractérisée en ce que la base est choisie parmi les acides aminés.

16. Emulsion selon l'une quelconque des revendications 9 à 15, caractérisée en ce que la base est choisie parmi la lysine et l'arginine.

17. Emulsion selon l'une quelconque des revendications 9 à 16, caractérisée en ce que le dérivé représente de 0,5 % à 10 % du poids total de l'émulsion.

18. Emulsion selon l'une quelconque des revendications 9 à 17, caractérisée en ce qu'elle contient au moins une huile polaire.

19. Emulsion selon la revendication 18, caractérisée en ce que l'huile représente de 10 % à 70 % du poids total de l'émulsion.

20. Emulsion selon l'une quelconque des revendications 9 à 19, caractérisée en ce qu'elle contient, en outre, au moins un adjuvant choisi parmi les céramides, les pseudocéramides, les glycocéramides et les acides gras essentiels.

21. Emulsion selon l'une quelconque des revendications 9 à 20, caractérisée en ce qu'elle contient, de plus, au moins un gélifiant.

22. Utilisation de l'émulsion selon l'une quelconque des revendications 9 à 21 pour le traitement non thérapeutique de la peau.

23. Utilisation de l'émulsion selon l'une quelconque des revendications 9 à 21 pour prévenir et/ou lutter contre l'acné et/ou la peau grasse et/ou le vieillissement et/ou la pigmentation de la peau, de façon non thérapeutique.

24. Procédé de traitement cosmétique de la peau pour lutter contre l'acné et/ou la peau grasse et/ou le vieillissement et/ou la pigmentation de la peau, consistant à appliquer sur celle-ci l'émulsion selon l'une quelconque des revendications 9 à 21.

## Claims

1. Use of a salicylic acid derivative salified with a base, as an agent for dispersing an oily phase in an aqueous phase in order to obtain an oil-in-water emulsion, free of surfactant, this derivative being situated at the oil/water interface.

2. Use according to Claim 1, characterized in that the derivative has the following formula (I): 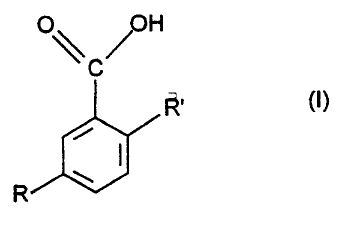 in which:
R represents a saturated, linear, branched or cyclized aliphatic, alkoxy, ester or ketoxy chain, or an unsaturated chain bearing one or more conjugated or non-conjugated double bonds, these chains containing from 2 to 22 carbon atoms and being able to be substituted with at least one substituent chosen from halogen atoms, the trifluoromethyl group, hydroxyl groups in free form or esterified with an acid having from 1 to 6 carbon atoms or alternatively with a carboxyl function, which is free or esterified with a lower alcohol having from 1 to 6 carbon atoms;
R' represents a hydroxyl group or an ester function of formula: 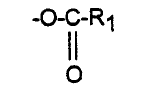 where R₁ is a saturated or unsaturated aliphatic group having from 1 to 18 carbon atoms.

3. Use according to Claim 2, characterized in that the radical R is an alkyl or alkoxy radical having from 4 to 11 carbon atoms.

4. Use according to any one of the preceding claims, characterized in that the salicylic acid derivative is n-octanoyl-5-salicylic acid.

5. Use according to any one of the preceding claims, characterized in that the base is an organic base.

6. Use according to any one of the preceding claims, characterized in that the base is an amphoteric base.

7. Use according to any one of the preceding claims, characterized in that the base is chosen from amino acids.

8. Use according to any one of the preceding claims, characterized in that the base is chosen from lysine and arginine.

9. Cosmetic and/or dermatological emulsion, characterized in that it contains at least one salicylic acid derivative of formula (I) below, salified with a base: 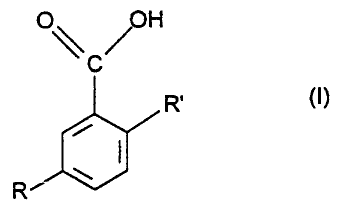 in which:
R represents a saturated, linear, branched or cyclized aliphatic, alkoxy, ester or ketoxy chain, or an unsaturated chain bearing one or more conjugated or non-conjugated double bonds, these chains containing from 2 to 22 carbon atoms and being able to be substituted with at least one substituent chosen from halogen atoms, the trifluoromethyl group, hydroxyl groups in free form or esterified with an acid having from 1 to 6 carbon atoms or alternatively with a carboxyl function, which is free or esterified with a lower alcohol having from 1 to 6 carbon atoms;
R' represents a hydroxyl group or an ester function of formula: 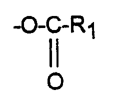 where R₁ is a saturated or unsaturated aliphatic group having from 1 to 18 carbon atoms,
this derivative being situated at the oil/water interface.

10. Emulsion according to Claim 9, characterized in that it is free of surfactant.

11. Emulsion according to Claim 9 or 10, characterized in that the radical R is an alkyl or alkoxy radical having from 4 to 11 carbon atoms.

12. Emulsion according to one of Claims 9 to 11, characterized in that the salicylic acid derivative is n-octanoyl-5-salicylic acid.

13. Emulsion according to one of Claims 9 to 12, characterized in that the base is an organic base.

14. Emulsion according to any one of Claims 9 to 13, characterized in that the base is an amphoteric base.

15. Emulsion according to any one of Claims 9 to 14, characterized in that the base is chosen from amino acids.

16. Emulsion according to any one of Claims 9 to 15, characterized in that the base is chosen from lysine and arginine.

17. Emulsion according to any one of Claims 9 to 16, characterized in that the derivative represents from 0.5% to 10% of the total weight of the emulsion.

18. Emulsion according to any one of Claims 9 to 17, characterized in that it contains at least one polar oil.

19. Emulsion according to Claim 18, characterized in that the oil represents from 10% to 70% of the total weight of the emulsion.

20. Emulsion according to any one of Claims 9 to 19, characterized in that it also contains at least one adjuvant chosen from ceramides, pseudoceramides, glycoceramides and essential fatty acids.

21. Emulsion according to any one of Claims 9 to 20, characterized in that it contains, in addition, at least one gelling agent.

22. Use of the emulsion according to any one of Claims 9 to 21, for the non-therapeutic treatment of the skin.

23. Use of the emulsion according to any one of Claims 9 to 21, for preventing and/or combating acne and/or greasy skin and/or the ageing and/or pigmentation of the skin, in a non-therapeutic manner.

24. Cosmetic treatment process for the skin in order to combat acne and/or greasy skin and/or the ageing and/or pigmentation of the skin, which consists in applying the emulsion according to any one of Claims 9 to 21 to the skin.

## Patentansprüche

1. Verwendung eines Salicylsäurederivats, das durch eine Base in die Form eines Salzes überführt ist, als Mittel zum Dispergieren einer Ölphase in einer wässerigen Phase zur Herstellung einer Öl-in-Wasser-Emulsion, die keinen grenzflächenaktiven Stoff enthält, wobei sich das Derivat an der Grenzfläche Öl/Wasser befindet.

2. Verwendung nach Anspruch 1, dadurch gekennzeichnet, daß das Derivat der folgenden Formel (I) entspricht: 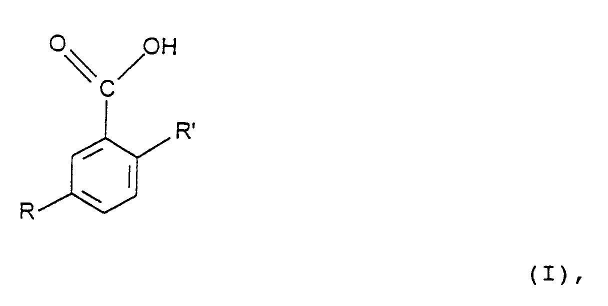 worin bedeuten:
- R eine gesättigte aliphatische Gruppe, Alkoxygruppe, Estergruppe oder Cetoxygruppe, die geradkettig, verzweigt oder cyclisch vorliegt, oder eine ungesättigte Gruppe, die eine oder mehrere gegebenenfalls konjugierte Doppelbindungen aufweist, wobei die Gruppen 2 bis 22 Kohlenstoffatome aufweisen und mit mindestens einem Substituenten substituiert sein können, der ausgewählt ist unter Halogen, Trifluormethyl, Hydroxy in freier Form oder verestert mit einer Säure, die 1 bis 6 Kohlenstoffatome aufweist, oder auch mit einer Carboxygruppe, die in freier Form oder mit einem niederen Alkohol mit 1 bis 6 Kohlenstoffatomen verestert vorliegt; und
- R' eine Hydroxygruppe oder eine Estergruppe der Formel: 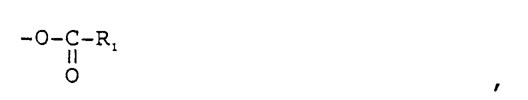 worin R₁ eine gesättigte oder ungesättigte aliphatische Gruppe mit 1 bis 18 Kohlenstoffatomen bedeutet.

3. Verwendung nach Anspruch 2, dadurch gekennzeichnet, daß die Gruppe R eine Alkylgruppe oder Alkoxygruppe mit 4 bis 11 Kohlenstoffatomen ist.

4. Verwendung nach einem der vorhergehenden Ansprüche, dadurch gekennzeichnet, daß das Salicylsäurederivat 5-(n-Octanoyl)-salicylsäure ist.

5. Verwendung nach einem der vorhergehenden Ansprüche, dadurch gekennzeichnet, daß die Base eine organische Base ist.

6. Verwendung nach einem der vorhergehenden Ansprüche, dadurch gekennzeichnet, daß die Base eine amphotere Base ist.

7. Verwendung nach einem der vorhergehenden Ansprüche, dadurch gekennzeichnet, daß die Base unter den Aminosäuren ausgewählt ist.

8. Verwendung nach einem der vorhergehenden Ansprüche, dadurch gekennzeichnet, daß die Base unter Lysin oder Arginin ausgewählt ist.

9. Kosmetische und/oder dermatologische Emulsion, dadurch gekennzeichnet, daß sie mindestens ein Salicylsäurederivat der nachfolgenden Formel (I) enthält, das durch eine Base in die Form eines Salzes überführt wurde: 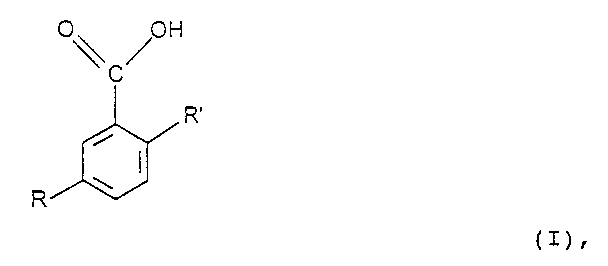 worin bedeuten:
- R eine gesättigte aliphatische Gruppe, Alkoxygruppe, Estergruppe oder Cetoxygruppe, die geradkettig, verzweigt oder cyclisch vorliegt, oder eine ungesättigte Gruppe, die eine oder mehrere gegebenenfalls konjugierte Doppelbindungen aufweist, wobei die Gruppen 2 bis 22 Kohlenstoffatome aufweisen und mit mindestens einem Substituenten substituiert sein können, der ausgewählt ist unter Halogen, Trifluormethyl, Hydroxy in freier Form oder verestert mit einer Säure, die 1 bis 6 Kohlenstoffatome aufweist, oder auch mit einer Carboxygruppe, die in freier Form oder mit einem niederen Alkohol mit 1 bis 6 Kohlenstoffatomen verestert vorliegt; und
- R' eine Hydroxygruppe oder eine Estergruppe der Formel: 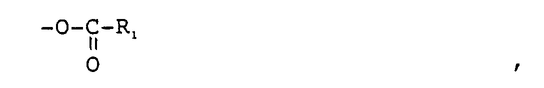 worin R₁ eine gesättigte oder ungesättigte aliphatische Gruppe mit 1 bis 18 Kohlenstoffatomen bedeutet, wobei sich das Derivat an der Grenzfläche Öl/Wasser befindet.

10. Emulsion nach Anspruch 9, dadurch gekennzeichnet, daß sie keinen grenzflächenaktiven Stoff enthält.

11. Emulsion nach Anspruch 9 oder 10, dadurch gekennzeichnet, daß die Gruppe R eine Alkylgruppe oder Alkoxygruppe mit 4 bis 11 Kohlenstoffatomen ist.

12. Emulsion nach einem der Ansprüche 9 bis 11, dadurch gekennzeichnet, daß das Salicylsäurederivat 5-(n-Octanoyl)-salicylsäure ist.

13. Emulsion nach einem der Ansprüche 9 bis 12, dadurch gekennzeichnet, daß die Base eine organische Base ist.

14. Emulsion nach einem der Ansprüche 9 bis 13, dadurch gekennzeichnet, daß die Base eine amphotere Base ist.

15. Emulsion nach einem der Ansprüche 9 bis 14, dadurch gekennzeichnet, daß die Base unter den Aminosäuren ausgewählt ist.

16. Emulsion nach einem der Ansprüche 9 bis 15, dadurch gekennzeichnet, daß die Base unter Lysin und Arginin ausgewählt ist.

17. Emulsion nach einem der Ansprüche 9 bis 16, dadurch gekennzeichnet, daß das Derivat 0,5 bis 10 % des Gesamtgewichts der Emulsion ausmacht.

18. Emulsion nach einem der Ansprüche 9 bis 17, dadurch gekennzeichnet, daß sie mindestens ein polares Öl enthält.

19. Emulsion nach Anspruch 18, dadurch gekennzeichnet, daß das Öl 10 bis 70 % des Gesamtgewichts der Emulsion ausmacht.

20. Emulsion nach einem der Ansprüche 9 bis 19, dadurch gekennzeichnet, daß sie ferner mindestens einen Zusatzstoff enthält, der unter den Ceramiden, Pseudoceramiden, Glykoceramiden und essentiellen Fettsäuren ausgewählt ist.

21. Emulsion nach einem der Ansprüche 9 bis 20, dadurch gekennzeichnet, daß sie ferner mindestens einen Gelbildner enthält.

22. Verwendung der Emulsion nach einem der Ansprüche 9 bis 21 zur nicht therapeutischen Behandlung der Haut.

23. Verwendung der Emulsion nach einem der Ansprüche 9 bis 21 zur nicht therapeutischen Vorbeugung und/oder Bekämpfung von Akne und/oder fettiger Haut und/oder der Alterung und/oder der Pigmentierung der Haut.

24. Verfahren zur kosmetischen Behandlung der Haut zur Bekämpfung von Akne und/oder fettiger Haut und/oder der Alterung und/oder der Pigmentierung der Haut, das darin besteht, die Emulsion nach einem der Ansprüche 9 bis 21 auf die Haut aufzutragen.
